# EUROPÄISCHE PATENTSCHRIFT

(11) **EP 1 863 557 B1**
(45) Veröffentlichungstag und Bekanntmachung des Hinweises auf die Patenterteilung: **01.12.2010**
(21) Anmeldenummer: 06722592.0
(22) Anmeldetag: 13.03.2006
(51) Int. Cl.: A61M 25/00

(54) **SYSTEM ZUM ZUFÜHREN EINES MITTELS IN EIN BLUTGEFÄSS**
SYSTEM FOR INTRODUCING AN AGENT INTO A BLOOD VESSEL
SYSTEME DESTINE A INTRODUIRE UN AGENT DANS UN VAISSEAU SANGUIN

(30) Priorität: 14.03.2005 DE 102005011656
(43) Veröffentlichungstag der Anmeldung: 12.12.2007
(73) Patentinhaber: Oxira Medical Inc., Boca Raton, FL 33433 (US)
(72) Erfinder: GEIS, John S., 26160 Bad Zwischenahn (DE); BRAUN, Michael, 71522 Backnang (DE)
(74) Vertreter: Cullinane, Marietta Bettina
(86) Internationale Anmeldenummer: PCT/DE2006/000435
(87) Internationale Veröffentlichungsnummer: WO 2006/097075

(56) Entgegenhaltungen:
- EP-A- 0 212 213
- WO-A-94/05361
- US-A1- 2004 022 824

## Beschreibung

Die vorliegende Erfindung betrifft ein System zum Zuführen eines Mittels in ein Blutgefäß und insbesondere ein cerebrales Oxygenierungssystem.

Infarkte stellen Ereignisse dar, bei denen es infolge einer Sauerstoffunterversorgung durch unzureichenden Blutfluss zu einem Gewebeuntergang, einer so genannten Nekrose, kommt. Meist entsteht ein Infarkt aufgrund eines vollständigen oder teilweisen Gefäßverschluss. Diese Gefäßverschlüsse können durch Blutgerinnsel, die auch als Thromben bezeichnet werden, oder durch anderes mit dem Blut eingeschwemmtes Material (Kalk, Gewebeteile oder Fettablagerungen) hervorgerufen werden.

Durch den gehinderten oder unterbrochenen Blutfluss zu dem in Blutflussrichtung hinter dem Gefäßverschluss liegenden Gewebe und weiteren Gefäßen, sterben Zellen und ganze Gewebeabschnitte schnell ab und es kommt zum Versagen der entsprechenden Funktionen. Beim Herzinfarkt oder Myokardinfarkt wird beispielsweise das Herzmuskelgewebe aufgrund des Sauerstoffmangels und dadurch hervorgerufenen Stoffwechselstörungen der Zellen zerstört. Bei einem Schlaganfall oder Apoplex nehmen die Hirnnervenzellen und das Zellgewebe rasch einen Schaden oder sterben ab. Es kommt auch hier aufgrund des Sauerstoffmangels und die dadurch entstandenen Stoffwechselstörungen zu einer Unterversorgung und einem Funktionsverlust eines Teils der Gehirns.

Zur Behandlung des unterversorgten Bereiches hinter dem Verschluss, der auch als ischämischer Bereich bezeichnet wird, muss den Zellen in diesem Bereich eine große Menge an Sauerstoff zur Verfügung gestellt werden. Bislang wird hierzu eine systemische Behandlung angewendet. Hierbei wird dem Patienten Sauerstoff, beispielsweise über eine Maske dargereicht. Durch die Einatmung kommt es zwar zu einer 100%igen Aufnahme des Sauerstoffs in dem Blut; aufgrund des vorliegenden Verschlusses wird aber nicht die erforderliche Menge an Sauerstoff zu den Zellen des Blutgefässes in dem ischämischen Bereich geleitet. Lokal wird somit die erforderliche Sättigung mit Sauerstoff, die zur Vermeidung des Absterbens der Zellen erforderlich wäre, nicht hinreichend erzielt.

Zur Zuführung von Medikamenten sind Vorrichtungen bekannt, bei denen eine Polymermatrix als Teil eines Katheters oder als Ummantelung eines Drahtes verwendet wird, über die die Medikamente abgegeben werden können. Solche Vorrichtungen sind beispielsweise in der WO 94/05361 A und US 2004/0022824 A1 beschrieben, Letztere Druckschrift offenbart die Merkmale des Oberbegriffs von Anspruch 1. Ein Nachteil dieser Vorrichtungen besteht darin, dass diese aufgrund ihrer Abmessungen und/oder der Anordnung der Polymermatrix nicht dazu geeignet sind, einen ischämischen Bereich eines Blutgefässes zuverlässig mit Sauerstoff zu versorgen.

Aufgabe der vorliegenden Erfindung ist somit, ein System zu schaffen, mit dem es möglich wird Bereiche eines Blutgefäßes, insbesondere einen ischämischen Bereich schnell mit einem Mittel, insbesondere mit einem Mittel zur Bereitstellung einer erforderlichen Menge an Sauerstoff, zu versorgen.

Der Erfindung liegt die Erkenntnis zugrunde, dass diese Aufgabe gelöst werden kann, indem ein System verwendet wird, bei dem eine Matrix für die Abgabe des Mittels im Inneren eines stabilen Zuführungssystem angeordnet ist.

Insbesondere wird die Aufgabe gelöst durch ein System zum Zuführen eines Mittels in ein Blutgefäß, wobei das System einen Draht mit einem proximalen Ende und einem distalen Ende umfasst. Das System zeichnet sich dadurch aus, dass in dem Draht zwischen dem proximalen und dem distalen Ende ein Lumen verläuft und in dem Lumen eine Matrix zur Abgabe des Mittels vorgesehen ist, wobei sich diese über zumindest einen Teil der Länge des Drahtes erstreckt.

Die Zuführung des Mittels erfolgt mit dem erfindungsgemäßen System vorzugsweise in den Blutstrom. Insbesondere erfolgt die Zuführung in einen teilweise oder vollständig verschossenen ischämischen Bereich eines Blutgefäßes. Das Mittel, das in diesen Bereich gebracht werden soll, kann in der erfindungsgemäß vorgesehenen Matrix aufgenommen sein und durch unterschiedliche Abgabemechanismen aus dieser abgegeben werden. Insbesondere kann das Mittel durch lokale Diffusion, durch Druck und/oder durch Temperaturänderung in den ischämischen Bereich abgegeben werden. Die Matrix fungiert somit als Substrat beziehungsweise Träger für das in das Blutgefäß einzubringende Mittel.

Durch die Kombination eines Drahtes mit einer Abgabematrix kann ein synergetischer Effekt erzielt werden.

Zum einen liefert die Verwendung eines Drahtes als Halter für die Abgabematrix eine stabile Anordnung. Der Draht ist vorzugsweise aus einem Metall oder einer Metalllegierung gefertigt. Besonders bevorzugt werden Nickel-Titan-Legierungen, wie Nitinol, Edelstahl und/oder Platin als Material verwendet. Aufgrund der Stabilität kann mit dem erfindungsgemäßen System ein Gefäßverschluss durchstoßen werden. Auf diese Weise wird ein Zugang zu dem ischämischen Bereich des Blutgefäßes geschaffen. Ein solches Durchstoßen kann mit einem flexiblen System, wie beispielsweise einem Kunststoffschlauch, nicht realisiert werden. Zudem kann bei der Verwendung eines Drahtes die Stabilität auch bei geringem Durchmesser des Drahtes bereit gestellt werden. Dieser geringe Durchmesser ist insbesondere zur Behandlung von Schlaganfällen von Bedeutung, da hierbei der Gefäßverschluss in Gefäßen auftritt, die einen sehr geringen Innendurchmesser aufweisen. Die cerebralen Gefäße können einen Durchmesser von 1,5 mm und weniger aufweisen.

Zum anderen ist das Mittel, das dem Bereich des Blutgefäßes zugeführt werden soll, in der innen liegenden Abgabematrix gehalten. Indem die Matrix im Inneren des Drahtes angeordnet ist, ist der äußere Durchmesser des Systems dadurch nicht beeinflusst und die Matrix wird beim Durchstoßen des Gefäßverschlusses auch nicht beschädigt. Das Vorsehen einer Matrix für die Abgabe des Mittels beeinflusst zudem die Qualität und Quantität des dadurch abgegebenen Mittels. Im Gegensatz zur Zuführung eines Mittels in ein Blutgefäß über ein Lumen eines Drahtes oder Katheters ohne Matrix, kann die Abgabe durch die Matrix gesteuert beziehungsweise dosiert werden. Insbesondere kann bei einem flüssigen Mittel ein Heraustropfen oder Herausschießen des Mittels aus dem Lumen verhindert werden. Auch die Qualität der Abgabe des Mittels wird durch das Vorsehen einer Matrix erhöht. Durch die Matrix kann insbesondere ein flüssiges Mittel verstäubt werden, das heißt die Tröpfchengröße des abgegebenen Mittels minimiert werden. Die Tropfengröße kann beispielsweise von 1µm - 300µm variiert werden. Die Matrix ist vorzugsweise so in dem Lumen des Drahtes vorgesehen, dass diese den gesamten inneren Durchmesser des Drahtes ausfüllt.

Weiterhin werden durch die Kombination eines Drahtes und einer darin aufgenommen Matrix auch Vorteile gegenüber Systemen erzielt, bei denen ein Katheter, durch den das Mittel eingebracht werden soll, über einen Führungsdraht geführt wird. Zwar kann bei einem solchen System der Führungsdraht zum Durchstoßen des Gefäßverschlusses dienen. Die Zuführung des Mittels muss dann aber über das Lumen des Katheters erfolgen. Hierbei kann das Mittel ausschließlich durch den Durchlass, der durch das Durchstoßen des Verschlusses durch den Führungsdraht in dem Verschluss geschaffen wurde, in den ischämischen Bereich gelangen, was in ausreichender Form mit therapeutischer Wirkung nahezu unmöglich ist. Weiterhin stellt die Zeit, die benötigt wird, den Katheter über den Führungsdraht zu leiten, einen Zeitverlust dar, der insbesondere bei der Behandlung von Schlaganfällen kritisch sein kann. Mit dem erfindungsgemäßen System hingegen wird, sobald der Draht in den ischämischen Bereich geführt wurde, eine Abgabe des Mittels ermöglicht.

Die Matrix ist in dem erfindungsgemäßen System vorzugsweise im Bereich des distalen Endes des Drahtes angeordnet. Das distale Ende des Drahtes, insbesondere die distale Öffnung, die das Ende des Lumens darstellt, ist die Stelle, an der das Lumen mit dem ischämischen Bereich in Kontakt steht. Die eingebundene Matrix in diesem Bereich wird erfindungsgemäß in das Lumen zu einem gewissen Teil eingebracht und kann mit dem Draht verbunden, beispielsweise verklebt werden. So wird zum einen ein Eintreten von Blut in das Lumen weitestgehend verhindert und zum anderen ein unkontrolliertes Austreten des Mittels aus dem Lumen in den ischämischen Bereich unterbunden.

Es ist erfindungsgemäß möglich, dass die Matrix ausschließlich im distalen Bereich des Drahtes vorgesehen ist. Hierdurch wird weniger Material zur

Herstellung der Matrix benötigt. Der verbleibende freie Bereich des Lumens kann mit einem abzugebenden flüssigen Mittel gefüllt werden. Somit kann in das Lumen eine große Menge des Mittels aufgenommen werden und für die Abgabe bereit stehen. Lediglich im distalen Bereich, in dem die Abgabe erfolgt, wirkt dann die Matrix zur Optimierung der Qualität und Quantität des abgegebenen Mittels.

Es ist erfindungsgemäß aber auch möglich die Matrix über die gesamte Länge des Drahtes, das heißt von dem distalen Ende bis zu dem proximalen Ende und gegebenenfalls auch über die Enden hinaus vorzusehen.

Diese Ausführungsform weist den Vorteil auf, dass die Matrix über die gesamte Länge des Drahtes als Leitungsmedium für das Mittel dienen kann. Insofern kann gemäß dem Dochtprinzip eine kontinuierliche Abgabe des Mittels durch Diffusion an dem distalen Ende des Drahtes durch Diffusion innerhalb der Matrix gewährleistet werden.

Gemäß einer Ausführungsform weist der Draht in dem distalen Bereich einen Abgabebereich auf, in dem mindestens eine Abgabeöffnung in der Drahtwand vorgesehen ist. Diese mindestens eine Abgabeöffnung erstreckt sich zwischen dem Innendurchmesser und dem Außendurchmesser des Drahtes. Durch die Öffnungen können somit zusätzlich zu der distalen Öffnung des Lumens weitere Abgabepunkte geschaffen werden und dadurch die zur Verfügung stehende Abgabefläche vergrößert werden. Die Öffnungen können beispielsweise Rundlöcher darstellen, die durch Laserbehandlung in die Wand des Drahtes eingebracht wurden.

Es ist aber auch möglich die mindestens eine Öffnung als Spiralöffnung in der Drahtwand vorzusehen. Somit erhält der distale Bereich des Drahtes die Form einer Spirale. Die Abgabefläche ist bei einer Spirale im Vergleich zu der Abgabefläche bei Löchern größer. Zudem wird auf dieser Weise dem distalen Bereich des Drahtes eine größere Flexibilität verliehen, wodurch das Einführen des Drahtes in ein Blutgefäß erleichtert wird und das Verletzungsrisiko des Patienten verringert wird.

Die Länge des Abgabebereichs, in dem die mindestens eine Abgabeöffnung vorgesehen ist, ist im Verhältnis zu der Länge des Gesamtdrahtes gering. Die Länge des Drahtes kann je nach Anwendung im Bereich von 30mm bis 3000mm liegen, während der Abgabebereich vorzugsweise eine Länge von 10mm bis 100mm aufweist.

Wird als Abgabeöffnung eine spiralförmige Öffnung verwendet, so kann der Abgabebereich mit dem weiteren Bereich des Drahtes integral ausgestaltet sein. Es ist aber auch möglich zur Herstellung des erfindungsgemäßen Drahtes die Spirale separat zu fertigen und mit einem Hohldraht zu verbinden, das heißt an dessen distalen Ende zu befestigen. Hierdurch wird die Herstellung des Drahtes vereinfacht. Dies ist zudem vorteilhaft, da bei der separaten Herstellung die Spirale aus einem anderen Material bestehen kann als der Hohldraht. So kann die Spirale beispielsweise aus Platin bestehen, während der Hohldraht aus Nitinol oder Edelstahl gefertigt ist. Die Platinspirale kann bei einem solchen System als Marker dienen, der dem operierenden Arzt eine bessere Navigation ermöglicht. Ein separater Marker muss in diesem Fall nicht vorgesehen werden. Zudem kann das System kostengünstig hergestellt werden, da der lange Hohldraht aus einem verhältnismäßig günstigerem Material gefertigt werden kann. Die Verbindung der Spirale mit dem Hohldraht zur Bildung des erfindungsgemäßen Drahtes kann durch Schweißen oder Löten erfolgen.

Besonders bevorzugt weist bei dieser Ausführungsform der Hohldraht an seinem distalen Ende einen Steg auf, der sich von dem distalen Ende des Lumens bis zu dem distalen Ende der Spirale erstreckt. Das distale Ende der Spirale kann dabei an dem distalen Ende des Steges befestigt werden. Zusätzlich kann die Spirale an dem distalen Ende des Hohldrahtes oder im distalen Bereich des Hohldrahtes fixiert und/oder befestigt werden.

Weiterhin ist es möglich den Abgabebereich durch einen Schlauch, insbesondere einen Kunststoffschlauch, z.B. PTFE-Schlauch, zu realisieren, in dessen Mantelflächen Löcher eingebracht sind und der auf das distale Ende eines Hohldrahtes aufgebracht wird und mit diesem verbunden wird. Der Abgabebereich wird bei dieser Ausgestaltung durch den, über den Hohldraht hinaus stehenden, Teil des Kunststoffschlauches, in dem Löcher in den Schlauchwänden vorgesehen sind, gebildet. Der Schlauch kann an der Außenseite des Hohldrahtes im distalen Bereich beispielsweise durch Ankleben befestigt werden.

Bei der Ausgestaltung des Abgabebereiches als Spirale oder bei der Verwendung von Löchern in dem Abgabebereich wird die Matrix vorzugsweise so angeordnet, dass diese die Löcher und/oder die Spiralöffnung vollständig abdeckt. Hierdurch wird eine größtmögliche Abgabefläche geschaffen. Wesentlich ist für die Anordnung der Matrix in dem Lumen des Drahtes allerdings nur, dass diese den Übergang zu dem Abgabebereich abdeckt, um ein Austreten des Mittels aus dem Lumen ohne Matrixstruktur zu verhindern.

Als Material für die Matrix wird vorzugsweise ein poröses Material, insbesondere ein poröser Kunststoff, verwendet. In den Poren kann das Mittel aufgenommen werden. Besonders bevorzugt wird Polytetrafluoretyhlen (PTFE), insbesondere ein expandiertes Polytetrafluoretyhlen (EPTFE) verwendet. Die Porengröße in der Matrix kann erfindungsgemäß im Bereich von 1µm bis 200µm liegen. Mit einer solchen Matrix kann eine kontinuierliche Abgabe des Mittels erzielt werden.

Die Matrix kann durch Einschäumen in das Lumen des Drahtes eingebracht werden. Bevorzugt wird die Matrix aber vorgefertigt und liegt in Form eines Fadens / Monofilamentes vor, der / das in das Lumen geführt wird.

Das Mittel, das mit dem erfindungsgemäßen System abgegeben werden kann, ist vorzugsweise ein flüssiges Mittel. Besonders bevorzugt können beispielsweise ein flüssiger Sauerstoffträger und / oder flüssige Medikamente mit dem erfindungsgemäßen System abgegeben werden. Ein bevorzugtes Beispiel eines Sauerstoffträgers ist eine Perfluorkarbon-Lösung, die mit Sauerstoff gesättigt ist. Perfluorkarbone eignen sich insbesondere aufgrund ihrer niedrigen Oberflächenspannung und der hohen Löslichkeit für Sauerstoff für die Behandlung ischämischer Bereiche.

Der Außendurchmesser des Drahtes kann gemäß einer Ausführungsform zumindest über einen Bereich zwischen dem proximalen und dem distalen Ende abnehmen. Besonders bevorzugt ist lediglich das distale Ende des Drahtes konisch ausgestaltet. Die konische Form kann durch Anschleifen der Außenseite des Drahtes erzeugt werden. Durch die Verjüngung an der Spitze des Drahtes kann das Einbringen des Systems in das Blutgefäß vereinfacht werden.

Bei einem Draht der aus einem Hohldraht und einer daran angebrachten Spirale besteht, liegt die Verjüngung vorzugsweise am distalen Bereich des Hohldrahtes vor. Der Außendurchmesser der Spirale hingegen ist konstant. Durch die Verjüngung des distalen Endes des Hohldrahtes kann eine Spirale mit einem geringeren Durchmesser verwendet und auf einfache Weise, beispielsweise durch Aufschieben, an dem Hohldraht fixiert werden. Besonders bevorzugt wird der Außendurchmesser der Spirale entsprechend dem Außendurchmesser des Hohldrahtes an dessen zylindrischem Teil gewählt. Der Innendurchmesser der Spirale kann hingegen etwas größer als der Durchmesser des Lumens der Hohldrahtes gewählt werden. Hierdurch kann sich der Durchmesser der Matrix, die sich bis in den Spiralbereich erstreckt, von dem Durchmesser des Lumens zu dem Innendurchmesser der Spirale im Übergangsbereich zwischen Hohldraht und Abgabebereich aufweiten.

Bei dieser Ausgestaltung wird die Spirale auf den konischen Teil des Drahtes aufgeschoben bis das proximale Ende der Spirale an dem distalen Ende des zylindrischen Bereiches des Hohldrahtes anliegt. Dort kann die Spirale mit dem Hohldraht verbunden beziehungsweise an diesem befestigt werden. Auf diese Weise erhält der Draht einen über seine Gesamtlänge gleich bleibenden Außendurchmesser. Der Abgabebereich wird bei dieser Ausgestaltung durch den Teil der Spirale gebildet, die über das distale Ende des Hohldrahtes hinaus steht.

Zur zusätzlichen Fixierung der Spirale oder eines Kunststoffschlauches an dem Hohldraht kann der Hohldraht an seinem distalen Ende, an dem sich die distale Öffnung des Lumens befindet, einen Steg aufweisen. Dieser Steg kann durch Entfernen, beispielsweise Abschleifen eines Teils der Mantelfläche des Hohldrahtes an dessen distalen Ende erzeugt werden und ist somit ein integraler Bestandteil des Hohldrahtes. Das distale Ende des Steges kann als weiterer Befestigungspunkt für eine Spirale oder einen Kunststoffschlauch dienen. Hier kann eine Spirale beispielsweise mit dem Steg verschmolzen werden. Hierdurch wird eine Verletzungsgefahr für den Pateienten minimiert und das distale Ende kann daher auch als atraumatischer Drahtkopf bezeichnet werden.

Durch den Steg wird eine Befestigung der Spirale oder des Schlauches gewährleistet, die ein Ablösen dieser Teile von dem Hohldraht beim Einführen oder beim Entfernen aus dem Blutgefäß verhindert.

Das erfindungsgemäße System kann eine Verbindungsvorrichtung umfassen, die lösbar an dem proximalen Ende des Drahtes befestigt werden kann und zur Zuleitung des Mittels in das Lumen dient. Diese Verbindungsvorrichtung umfasst insbesondere ein Ventil, über das das proximale Ende des Drahtes in einen geschlossenen Behälter geführt werden kann, in dem sich das einzubringende Mittel befindet.

Weiterhin kann die Verbindungsvorrichtung zusätzlich oder alternativ einen Zugang für eine Spritze, einen so genannten Sideport, aufweisen. Dieser endet vorzugsweise in der Verbindungsvorrichtung hinter dem Ventil, das heißt in dem Hauptkanal des Ventils. Hierdurch kann mittels einer Spritze Druck in dem Lumen des Drahtes erzeugt werden.

Die Tatsache, dass die Verbindungsvorrichtung nur zeitweise mit dem Draht des Systems verbunden wird, weist einige Vorteile auf. Zum einen kann nach dem Lösen der Verbindungsvorrichtung von dem Draht, dieser für weitere Verwendungen zur Verfügung stehen. So kann der Draht des Systems beispielsweise als Führungsdraht für einen Mikrokatheter verwendet werden. Über diesen Katheter können Reagenzien für die Auflösung von Thromben bis vor die Verschlussstelle in dem Blutgefäß geleitet werden. Auch während der Katheter eingeführt wird kann die Abgabematrix weiterhin das Mittel hinter dem Verschluss in dem Blutgefäß abgeben. Der Draht des erfindungsgemäßen Systems kann zu diesem Zweck nach dem Entfernen der Verbindungsvorrichtung an dem proximalen Ende mit einem Endteil verschlossen werden. Dieses Endteil, das beispielsweise einen Pfropfen oder Deckel darstellen kann, verhindert ein Austreten des Mittels am proximalen Ende des Drahtes und dient zudem dazu Druckverhältnisse in dem Lumen des Drahtes aufrecht zu erhalten.

Es ist auch möglich den Draht im Bereich des proximalen Endes mit Aufnahmeöffnungen in der Drahtwand zu versehen. Hierdurch kann die Aufnahmefläche für ein einzubringendes Mittel erhöht werden. Auch diese Aufnahmeöffnungen können beispielsweise durch Laserbehandlung in die Drahtwand eingebracht sein. In diesem Fall wird zum Verschließen des proximalen Endes ein Endteil verwendet, das den proximalen Bereich, in dem Öffnungen vorgesehen sind, umschließt.

Die Erfindung wird im Folgenden anhand der beiliegenden Zeichnungen, die sich auf mögliche Ausführungsbeispiele der Erfindung beziehen, beschrieben. Es zeigen:
Figur 1: eine schematische Darstellung einer ersten Ausführungsform des erfindungsgemäßen Systems;
Figur 2: eine schematische Darstellung einer zweiten Ausführungsform des erfindungsgemäßen Systems;
Figur 3: eine schematische Darstellung einer dritten Ausführungsform des erfindungsgemäßen Systems;
Figur 4: eine schematische Frontansicht einer Ausführungsform des erfindungsgemäßen Systems;
Figur 5: eine schematische Detailansicht des Abgabebereiches einer bevorzugten Ausführungsform des erfindungsgemäßen Systems;
Figur 6: eine schematische Detailansicht des distalen Endes des Hohldrahtes gemäß der Ausführungsform des erfindungsgemäßen Systems nach Figur 5;
Figur 7: eine schematische Darstellung einer vierten Ausführungsform des erfindungsgemäßen Systems;
Figur 8: eine schematische Darstellung einer Ausführungsform einer Verbindungsvorrichtung des erfindungsgemäßen Zuführungssystems;
Figur 9: eine schematische Darstellung des erfindungsgemäßen Systems in einem Blutgefäß; und
Figur 10: eine schematische Detaildarstellung des Abgabebereiches eines erfindungsgemäßen Systems in einem Blutgefäß.

Die Darstellungen sind nicht maßstabsgetreu.

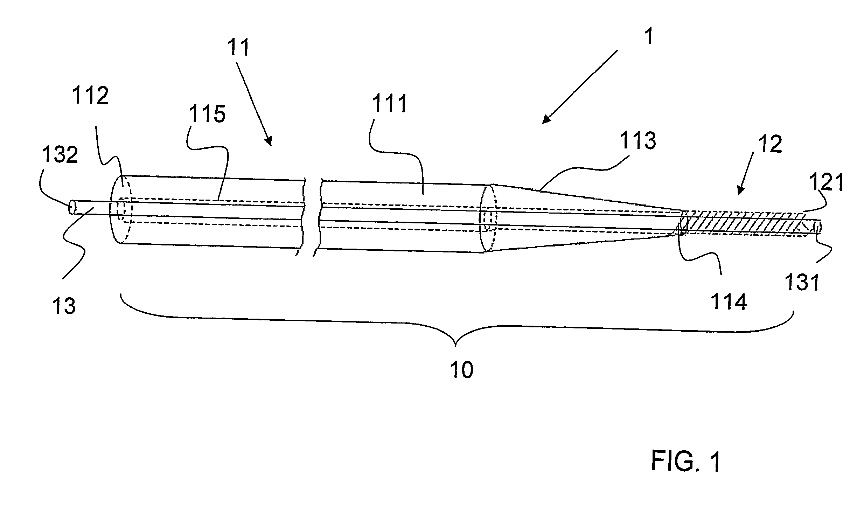

In Figur 1 ist eine Ausführungsform des erfindungsgemäßen Zuführungssystems 1 schematisch dargestellt. Das System 1 umfasst in dieser Ausführungsform einen Draht 10. Dieser besteht aus einem Hohldraht 11 und einer Spirale 12.

Der Hohldraht 11 weist einen zylindrischen Bereich 111 auf, der sich von dem proximalen Ende 112 des Hohldrahtes 11 erstreckt. Dieser Bereich 111 ist nur ausschnittsweise dargestellt. In einer Ausführungsform erstreckt sich dieser zylindrische Bereich 111 beispielsweise über 1500mm. An den zylindrischen Bereich 111 des Hohldrahtes 11 schließt sich ein konischer Bereich 113 an, in dem der Außendurchmesser des Hohldrahtes 11 abnimmt. An der distalen Spitze 114 besitzt der Hohldraht 11 damit einen geringen Außendurchmesser. Über den zylindrischen Bereich 111 und den konischen Bereich 113 hinweg erstreckt sich im Inneren ein Lumen 115 in dem Hohldraht 11. Der Durchmesser des Lumens 115, d.h. des Innendurchmessers des Hohldrahtes 11, ist über die Länge des Hohldrahtes 11 konstant.

An der distalen Spitze 114 des Hohldrahtes 11 schließt sich bei der dargestellten Ausführungsform eine Spirale 12 an, die an der Spitze 114 befestigt ist. Der Innendurchmesser der Spirale 12 stellt hierbei die Fortsetzung des Lumens 115 des Hohldrahtes 11 dar und weist vorzugsweise auch den gleichen Durchmesser wie das Lumen 115 auf.

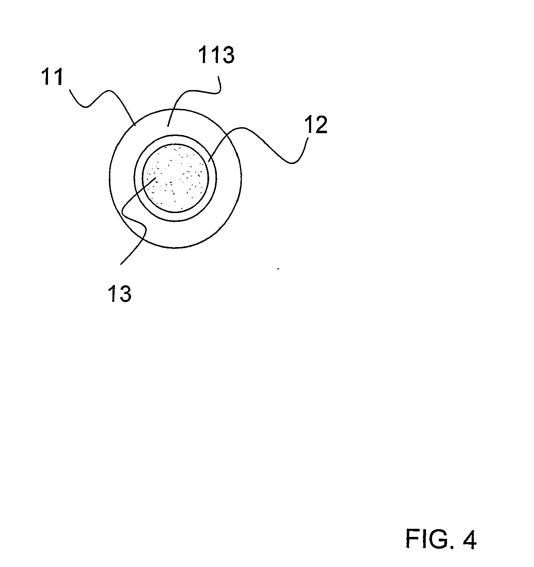

Durch das Lumen 115 des Hohldrahtes 11 und der Spirale 12 erstreckt sich in der dargestellten Ausführungsform eine Matrix 13. Dieses besteht in der dargestellten Ausführungsform aus einem Faden oder Monofilament aus ePTFE und dient der Abgabe eines Mittels, das durch diese geführt wird beziehungsweise in dieser aufgenommen ist. Die Matrix 13 füllt, wie sich aus Figur 4 ergibt, im wesentlichen den gesamten Querschnitt des Lumens 115 des Hohldrahtes 11 beziehungsweise der Spirale 12 aus. Im Bereich der Spirale 12 tritt die Matrix 13 an den Zwischenräumen zwischen den Spiralwindungen mit der Umgebung in Kontakt. Zudem steht auch das distale Ende 131 der Matrix 13 durch die Öffnung der Spirale 12 an deren distalen Ende 121 mit der Umgebung in Kontakt.

An dem proximalen Ende 112 des Hohldrahtes 11 ragt in der dargestellten Ausführungsform die Matrix 13 über den Hohldraht 11 hinaus. Die Matrix 13 kann aber auch bündig mit dem Ende 113 des Hohldrahtes 11 enden.

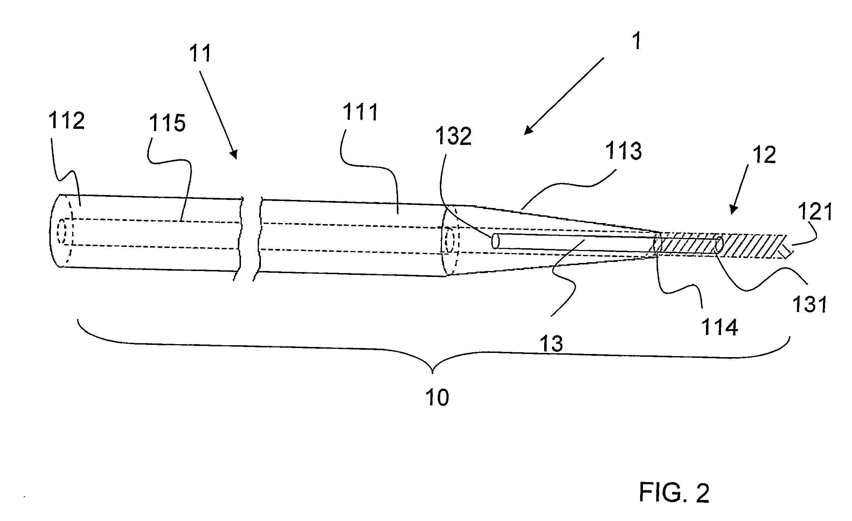

In der Figur 2 ist eine zweite Ausführungsform des erfindungsgemäßen Systems 1 gezeigt. Der Aufbau des Systems 1 entspricht im wesentlichen dem der ersten Ausführungsform. Die Bestandteile sind mit den entsprechenden Bezugsziffern bezeichnet und deren Funktion wird nicht erneut erläutert.

Im Unterschied zu der ersten Ausführungsform ist in der zweiten Ausführungsform die Matrix 13 allerdings nur über den Bereich der Spirale 12 und einem Teil des Lumens 115 des Hohldrahtes 11 vorgesehen. In der dargestellten Ausführungsform endet die Matrix 13 in einem Abstand zu der distalen Spitze 121 der Spirale 12. Es liegt aber auch im Rahmen der Erfindung, dass diese sich bis an die Spitze 121 der Spirale 12 erstreckt. Auch das proximale Ende 132 der Matrix 13 ist nicht auf die dargestellte Position im konischen Bereich 113 des Hohldrahtes 11 beschränkt. Die Matrix 13 kann auch im zylindrischen Teil 111 des Hohldrahtes 11 enden. Wesentlich ist lediglich, dass an der distalen Spitze 114 des Hohldrahtes 11 die Matrix 13 das Lumen 115 des Hohldrahtes 11 abdeckt.

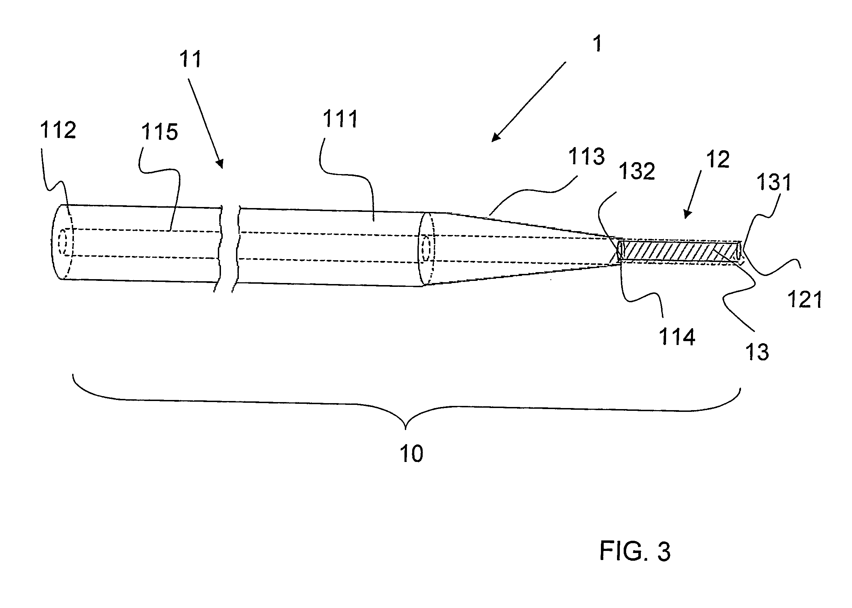

In Figur 3 ist eine dritte Ausführungsform des erfindungsgemäßen Zuführungssystems 1 gezeigt. Der Aufbau dieser Ausführungsform entspricht ebenfalls im wesentlichen der in Figur 1 gezeigten Ausführungsform. Allerdings ist in der dargestellten Ausführungsform die Matrix 13 ausschließlich im Bereich der Spirale 12 vorgesehen. Hierbei kann das distale Ende 131 der Matrix 13 von dem distalen Ende 121 der Spirale 12 beabstandet sein, das heißt die Matrix kann in der Spirale enden. Allerdings wird die Matrix 13 stets so angeordnet, dass deren proximales Ende 132 zumindest an der Spitze 114 des Hohldrahtes 11 oder sogar in dem Lumen 115 des Hohldrahtes 11 liegt.

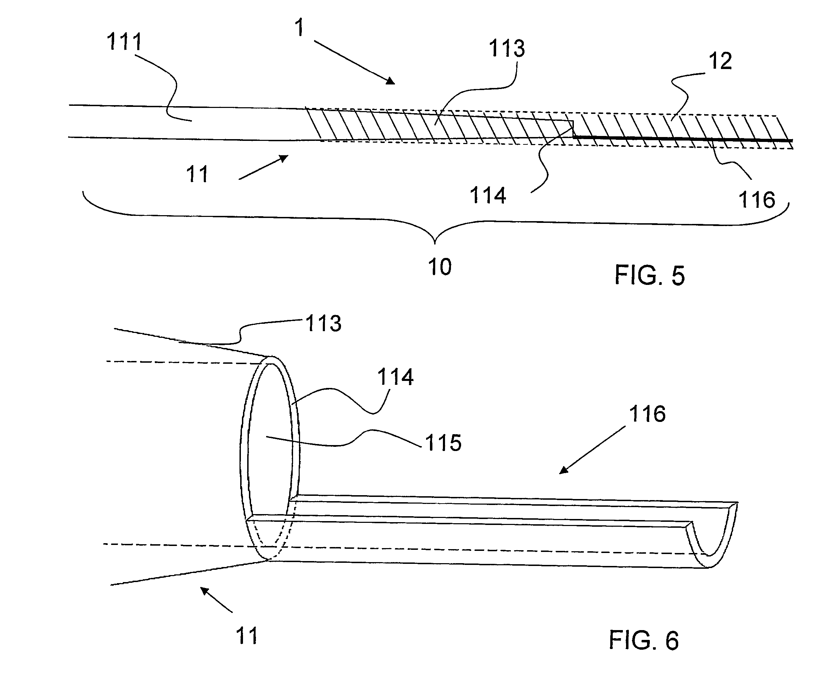

In Figur 5 ist eine bevorzugte Ausführungsform des Abgabebereiches des erfindungsgemäßen Systems 1 gezeigt. Bei dieser Ausführungsform weisen die Spirale 12 und der zylindrische Teil 111 des Hohldrahtes 11 den gleichen Außendurchmesser auf. Die Spirale 12 ist auf den Hohldraht 11 soweit aufgeschoben, dass diese im wesentlichen den gesamten konischen Bereich 113 des Hohldrahtes 11 überdeckt. Somit erhält der gesamte Draht 10 einen durchgehenden äußeren Durchmesser. Zusätzlich ist an dem Hohldraht 11 an dessen distalem Ende 114 ein Steg 116 vorgesehen, an dessen distalen Ende die Spirale 12 mit dem Steg 116 und dadurch mit dem Hohldraht 11 verbunden ist. Zusätzlich kann die Spirale 12 bei dieser Ausführungsform an dem Beginn des konischen Bereiches 113, das heißt an dem distalen Ende des zylindrischen Bereiches 111 befestigt sein.

Diese in Figur 5 gezeigte Art der Verbindung der Spirale 12 mit dem Hohldraht 11 wird vorzugsweise auch bei den in den Figuren 1 bis 3 dargestellten Ausführungsformen realisiert.

In Figur 6 ist die Detailansicht des distalen Endes 114 des Hohldrahtes 11 mit einem Steg 116 nach Figur 5 gezeigt. Aus dieser Ansicht lässt sich erkennen, dass der Steg 116 eine Fortführung der Außenwand des Hohldrahtes 11 darstellt.

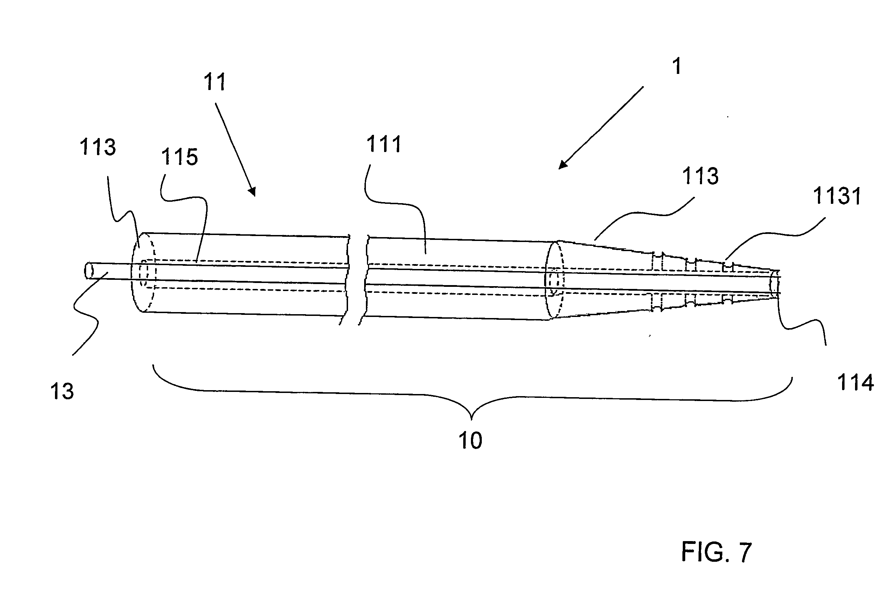

In Figur 7 ist eine vierte Ausführungsform des erfindungsgemäßen Systems 1 dargestellt. Hierbei umfasst der Draht 10 des Systems 1 lediglich einen Hohldraht 11. Dieser besitzt ebenfalls einen zylindrischen 111 und einen konischen Bereich 113. Im konischen Bereich 113 sind in der Nähe zu dem distalen Ende 114 des Drahtes 11 Öffnungen 1131 in Form von Löchern vorgesehen, die sich bis zu dem Lumen 115 des Hohldrahtes 11 erstrecken. In der dargestellten Ausführungsform sind die Öffnungen 1131 senkrecht zu der Achse des Hohldrahtes 11 angeordnet. Es liegt aber auch im Rahmen der Erfindung die Öffnungen 1131 unter einem anderen Winkel zu der Achse vorzusehen. Auch die Anzahl und Verteilung der Öffnungen 1131 sind nicht auf die dargestellte Ausführungsform beschränkt.

In dem Lumen 115 ist erneut eine Matrix 13 vorgesehen. Diese erstreckt sich in der dargestellten Ausführungsform über die gesamte Länge des Hohldrahtes 11. Es ist aber auch möglich die Matrix 13 nur in dem Bereich vorzusehen, in dem die Öffnungen 1131 in der Wand des Hohldrahtes 11 eingebracht sind.

Die Matrix 13 steht bei der vierten Ausführungsform somit an dem distalen Ende 114 des Hohldrahtes 11 und über die Öffnungen 1131 in der Wand des Hohldrahtes 11 mit der Umgebung in Kontakt.

Die Verwendung des erfindungsgemäßen Systems 1 und dessen Vorteile werden im Folgenden unter Bezugnahme auf die Zeichnungen erneut anhand des Beispiels der Behandlung eines Schlaganfall-Patienten erläutert. Aus Gründen der besseren Verständlichkeit wird hierbei soweit nichts anderes erwähnt ist, auf die erste Ausführungsform, die in Figur 1 gezeigt ist, Bezug genommen.

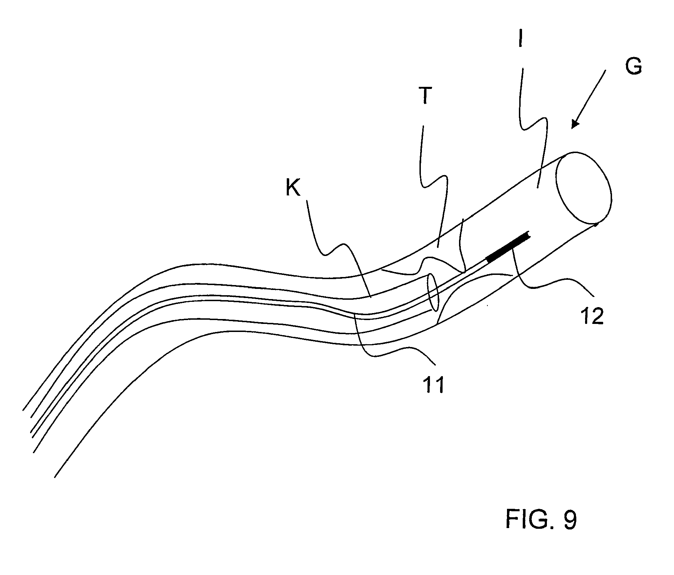

In Figur 9 ist ein Blutgefäß, insbesondere ein cerebrales Blutgefäß eines Schlaganfall-Patienten schematisch dargestellt. Das Blutgefäß G ist zumindest teilweise durch Thromben T verschlossen. Hierdurch wird der Bereich I, der in Blutflussrichtung hinter dem Verschluss T liegt, ischämisch.

In diesem Fall kann nun zunächst die Leiste des Patienten punktiert werden, insbesondere im Bereich der Arteria brachialis oder femoralis. Über diese Punktierung kann der Draht 10 des Systems 1 eingebracht und bis in die cerebralen Gefäße G geführt. Die an dem Hohldraht 11 vorgesehene Spirale 12, kann hierbei dem Chirurgen als Marker dienen, so dass dieser das Fortschreiten des Drahtes 10 gut verfolgen kann. Der Draht 10 wird bis zu dem durch die Thromben gebildeten Verschluss T vorgeschoben und durch diese hindurch gezwungen. Durch den Durchbruch des Verschlusses T gelangt der Bereich der Spirale 12 in den ischämischen Bereich I. In der dargestellten Position in Figur 9 ist der Draht 10 so weit in den ischämischen Bereich I geschoben, dass die gesamte Spirale 12 dort vorliegt.

Auf diese Weise kann bei Verwendung des erfindungsgemäßen Systems 1 schon bei noch bestehendem Verschluss T eine Oxygenierung der dahin liegenden Gefäße erfolgen.

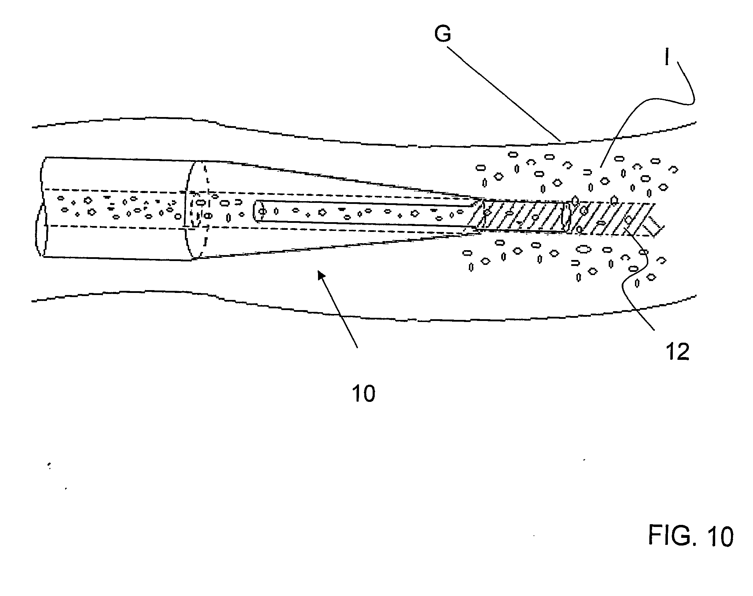

Der Austritt des Mittels aus dem erfindungsgemäßen System ist in Figur 10 angedeutet. Hierbei ist das Mittel durch die Punkte angedeutet.

Die zur Oxygenierung des ischämischen Bereiches I notwendige Befüllung des Systems 1 mit dem abzugebenden Mittel, kann bei dem erfindungsgemäßen System 1 nach der Ausführungsform der Figur 1 wie folgt durchgeführt werden. Bevor der Draht 10 in das Blutgefäß G bis in den ischämischen Bereich I geführt wird, wird dieser mit seinem proximalen Ende 112 in eine Verbindungsvorrichtung 14 eingeführt.

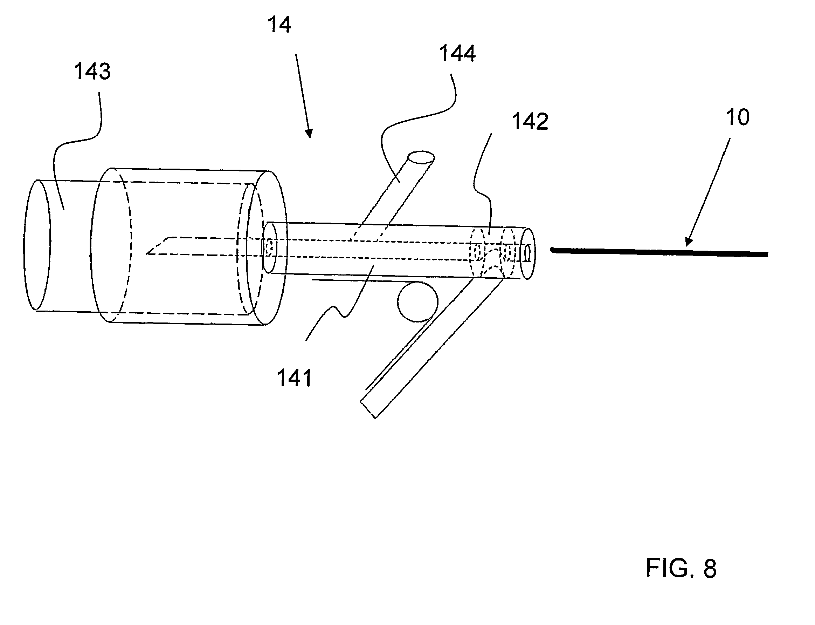

Eine solche Verbindungseinrichtung 14 ist beispielhaft in Figur 8 gezeigt. Das proximale Ende 112 des Drahtes 10 wird hierbei über einen Hauptkanal 141, in dem ein Ventil 142 vorgesehen ist, in einen Behälter 143 geführt, in dem sich das einzubringende Mittel befindet. Dieses Mittel kann beispielsweise ein flüssiger Sauerstoffträger oder eine Medikamentenlösung sein. Besonders bevorzugt wird eine Perfluorcarbon-Lösung verwendet, die mit Sauerstoff gesättigt ist.

Durch das Eintauchen des Drahtes 10 mit seinem proximalen Ende 112 in das Mittel, tritt die Matrix 13, die in dem Draht 11 des Systems 1 vorgesehen ist, mit dem Mittel in Kontakt. Das Mittel kann in die Matrix 13 eindringen und diese tränken. Zusätzlich oder alternativ kann insbesondere bei Ausführungsformen, bei denen sich die Matrix 13 nicht bis zum proximalen Ende 112 des Drahtes 10 erstreckt, über einen Zugang 144 an der Verbindungseinrichtung 14 beispielsweise durch eine Spritze (nicht dargestellt) Druck aufgebracht werden, wodurch das Mittel in das Lumen 115 und die Matrix 13 gedrückt wird.

Dieser aufgebrachten Druck sorgt dafür, dass das Mittel über die Matrix 13 an dem distalen Ende 114 des Hohldrahtes 11, das heißt im Bereich der Spirale 12, austreten kann. Der Druck wird so gering gehalten, dass ein Heraustropfen oder Herausspritzen über die Matrix 13 verhindert wird. Vorzugsweise wird nur ein anfänglicher Druck aufgebracht. Durch die in dem Lumen 115 vorgesehene Abgabematrix 13 wird das Mittel anschließend nach dem Docht-Prinzip zu der Spitze 114 des Hohldrahtes 11 und damit in den Bereich der Spirale 12 gesogen. Nach dem Befüllen oder Laden des Drahtes 10 mit dem Mittel kann somit die lösbar mit dem Draht 10 verbundene Verbindungsvorrichtung 14 entfernt werden. Statt des Ventils kann anschließend ein Endteil (nicht dargestellt) auf das proximale Ende 112 des Drahtes 10 zum Verschließen der proximalen Öffnung des Lumens 115 aufgesetzt werden, das ein Austreten des Mittels verhindert und die eingestellten Druckverhältnisse in dem Draht 10 aufrecht erhält.

Der Draht des erfindungsgemäßen Systems 1 kann als Führungsdraht oder Guidewire für einen Katheter K, insbesondere Mikrokatheter, verwendet werden. Der Katheter kann über den Draht 10 geführt werden, sobald dieser den ischämschen Bereich erreicht hat. Somit wird eine Oxygenierung des ischämischen Bereiches I bereits zu einem frühren Zeitpunkt ermöglicht. Der Mikrokatheter K wird bis vor den Verschluss beziehungsweise die Thromben T gebracht. Über den Mikrokatheter K wird eine arterielle Thrombolyse mit auflösenden Mitteln durchgeführt.

Die Erfindung ist nicht auf die dargestellten Ausführungsformen beschränkt.

Der Außendurchmesser des Drahtes oder Hohldrahtes kann an dessen größten Stelle im Bereich von 0,3mm bis 1mm und vorzugsweise im Bereich von 0,3 bis 0,4mm liegen. Der Durchmesser des Innenlumens der Drahtes oder Hohldrahtes kann im Bereich von 0,1 bis 0,3mm vorzugsweise im Bereich von 0,15 bis 0,25 mm und besonders bevorzugt bei 0,2mm liegen.

Die Spirale, die erfindungsgemäß an dem distalen Ende des Drahtes vorgesehen sein kann, ist vorzugsweise eine Platinspirale. Diese kann einen Außendurchmesser vom 0,3mm bis 1,0mm, vorzugsweise einen Außendurchmesser von 0,4mm aufweisen. Der Innendurchmesser kann im Bereich von 0,1mm bis 0,3mm, vorzugsweise bei 0,25mm liegen.

Die Matrix, das heißt das Substrat beziehungsweise der Träger für das abzugebende Mittel, kann erfindungsgemäß vorzugsweise aus ePTFE bestehen und Poren in der Größe von 1µm bis 200µm, vorzugsweise in der Größe von 80µm aufweisen. Der Durchmesser der Matrix kann im Bereich von 0,1 bis 0,3mm vorzugsweise im Bereich von 0,15 bis 0,25 und besonders bevorzugt bei 0,2mm liegen.

Gemäß einer Ausführungsform beträgt die Länge des Systems, insbesondere des Drahtes beziehungsweise Hohldrahtes, 1800mm, wobei dieser vom proximalen Ende aus 1500mm einen gleich bleibenden Durchmesser aufweist und an dem distalen Ende einen konischen Verlauf besitzt.

Mit dem erfindungsgemäßen System wird die Möglichkeit geschaffen Mittel, wie beispielsweise Sauerstoffträger, auf einfache Weise und schnell in einen Bereich eines Blutgefäßes zu liefern, der durch einen Gefäßverschluss begrenzt ist.

## Patentansprüche

1. System zum Zuführen eines Mittels in ein Blutgefäß (G), wobei das System (1) zumindest einen Draht (10) mit einem proximalen Ende (114, 121) und einem distalen Ende (112) sowie eine Matrix (13) umfasst, **dadurch gekennzeichnet, dass** in dem Draht (10) zwischen dem proximalen und dem distalen Ende ein Lumen (115) verläuft und in dem Lumen (115) die Matrix (13) zur Abgabe des Mittels vorgesehen ist, wobei sich diese über zumindest einen Teil der Länge des Drahtes (10) erstreckt.

2. System nach Anspruch 1, **dadurch gekennzeichnet, dass** die Matrix (13) im Bereich des distalen Endes (114, 121) des Drahtes (10) angeordnet ist.

3. System nach einem der Ansprüche 1 oder 2, **dadurch gekennzeichnet, dass** die Matrix (13) sich über die gesamte Länge des Drahtes (10) erstreckt.

4. System nach einem der Ansprüche 1 bis 3, **dadurch gekennzeichnet, dass** der Außendurchmesser des Drahtes (10) zu dem distalen Ende (114, 121) des Drahtes (10) abnimmt.

5. System nach einem der Ansprüche 1 bis 4, **dadurch gekennzeichnet, dass** der Draht (10) am distalen Ende einen Abgabebereich (12, 1131) aufweist, in dem in der Drahtwand mindestens eine Abgabeöffnung (1131) vorgesehen ist.

6. System nach Anspruch 5, **dadurch gekennzeichnet, dass** der Abgabebereich durch eine Spirale (12) gebildet wird.

7. System nach einem der Ansprüche 1 bis 6, **dadurch gekennzeichnet, dass** die Matrix (13) aus einem porösen Kunststoff, insbesondere ePTFE, besteht.

8. System nach einem der Ansprüche 1 bis 7, **dadurch gekennzeichnet, dass** dieses eine Verbindungsvorrichtung umfasst, die lösbar im Bereich des proximalen Endes (112) des Drahtes (10) befestigt werden kann und zur Zuleitung des Mittels in das Lumen (115) dient.

## Claims

1. System for introduction of an agent into a blood vessel (G), wherein the system (1) comprises at least a wire (10) having a proximal end (114, 121) and a distal end (112) as well as a matrix (13), **characterized in that** a lumen (115) extends in the wire (10) between the proximal end and the distal end and the matrix (13) for release of the agent is provided within the lumen (115), wherein the matrix (13) extends over at least part of the length of the wire (10).

2. System according to claim 1, **characterized in that** the matrix (13) is arranged in the area of the distal end (114, 121) of the wire (10).

3. System according to anyone of claims 1 or 2, **characterized in that** the matrix (13) extends over the entire length of the wire (10).

4. System according to anyone of claims 1 to 3, **characterized in that** the outer diameter of the wire (10) decreases towards the distal end (114, 121) of the wire (10).

5. System according to anyone of claims 1 to 4, **characterized in that** the wire (10) has a release region (12, 1131) at its distal end, wherein at least one release opening (1131) is provided in the wire wall.

6. System according to claim 5, **characterized in that** the release area is formed by a spiral (12).

7. System according to anyone of claims 1 to 6, **characterized in that** the matrix (13) consists of a porous plastic, in particular ePTFE.

8. System according to anyone of claims 1 to 7, **characterized in that** it comprises a connecting device, which can detachably be attached at the area of the proximal end (112) of the wire (10) and serves for introduction of the agent into the lumen (115).

## Revendications

1. Système destiné à introduire un agent dans un vaisseau sanguin (G), le système (1) comprenant au moins un fil (10) doté d'une extrémité proximale (114, 121) et d'une extrémité distale (112), et une matrice (13), **caractérisé en ce qu'**un lumen (115) évolue dans le fil (10) entre l'extrémité proximale et l'extrémité distale (115) et dans le lumen (115), la matrice (13) est prévue pour distribuer l'agent, celle-ci s'étendant sur au moins une partie de la longueur du fil (10).

2. Système selon la revendication 1, **caractérisé en ce que** la matrice (13) est disposée dans la zone de l'extrémité distale (114, 121) du fil (10).

3. Système selon l'une des revendications 1 ou 2, **caractérisé en ce que** la matrice (13) s'étend sur la longueur totale du fil (10).

4. Système selon l'une des revendications 1 à 3, **caractérisé en ce que** le diamètre externe du fil (10) diminue vers l'extrémité distale (114, 121) du fil (10).

5. Système selon l'une des revendications 1 à 4, **caractérisé en ce que** le fil (10) présente à l'extrémité distale, une zone de distribution (12, 1131) dans laquelle au moins une ouverture de distribution (1131) est prévue dans la paroi du fil.

6. Système selon la revendication 5, **caractérisé en ce que** la zone de distribution est formée par une spirale (12).

7. Système selon l'une des revendications 1 à 6, **caractérisé en ce que** la matrice (13) est constituée d'une matière plastique poreuse, en particulier d'ePTFE.

8. Système selon l'une des revendications 1 à 7, **caractérisé en ce que** celui-ci comprend un dispositif de raccordement qui peut être fixé de façon amovible dans la zone de l'extrémité proximale (112) du fil (10) et sert à acheminer l'agent dans le lumen (115).
